# EUROPÄISCHE PATENTANMELDUNG

(11) **EP 0 974 338 A2**
(43) Veröffentlichungstag der Anmeldung: **26.01.2000**
(21) Anmeldenummer: 99113498.2
(22) Anmeldetag: 13.07.1999
(51) Int. Cl.: A61K 7/48, A61K 7/06

(54) **Pflegende kosmetische und dermatologische Zubereitungen mit einem Gehalt an Fettsäuren**

(30) Priorität: 22.07.1998 DE 19832888
(71) Anmelder: Beiersdorf Aktiengesellschaft, 20245 Hamburg (DE)
(72) Erfinder: Lanzendörfer, Ghita, Dr., 22087 Hamburg (DE); Schreiner, Volker, Dr., 20259 Hamburg (DE); Schneider, Günther, Dr., 22607 Hamburg (DE); Wolf, Florian, Dr., 20251 Hamburg (DE)

(57) **Zusammenfassung**

Kosmetische oder dermatologische Zubereitungen mit einem Gehalt an unverzweigten, gesättigten und/oder ungesättigten, aliphatischen Fettsäuren, die eine Kettenlänge von C₂₂ bis C₆₀ haben, wobei die genannten Fettsäuren sowohl einzeln als auch im Gemisch vorliegen können.

## Beschreibung

Die vorliegende Erfindung betrifft Zubereitungen zur Pflege und Reinigung trockener und beanspruchter Haut und Altershaut sowie zur Behandlung und Prophylaxe von Folgeschäden der Hautaustrocknung, wie sie beispielsweise bei der Behandlung mit waschaktiven Substanzen auftreten können.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias (*P*. *M*. *Elias*, *Structure and Function of the Stratum Corneum Permeability Barrier*, *Drug Dev*. *Res*. ***13***, *1988*, *97-105*) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin und Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden offenbar fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionweise der Hornschicht entscheidend beeinträchtigt.

Unter Hautpflege im Sinne der vorliegenden Erfindung ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, Lipide, Elektrolyte) gestärkt oder wiederhergestellt wird.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im wesentlichen auf der Abdeckung (Okklusion) der behandelten Hautbezirke. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Maße erreicht.

Die Wirkung einiger pharmazeutischer Zubereitungen auf die Barrierefunktion der Haut besteht sogar in einer selektiven Barriereschädigung, die ermöglichen soll, daß Wirkstoffe in bzw. durch die Haut in den Körner eindringen können. Ein gestörtes Erscheinungsbild der Haut wird dabei als Nebenwirkung teilweise billigend in Kauf genommen.

Die Wirkung von pflegenden Reinigungsprodukten besteht im wesentlichen in einer effizienten Rückfettung mit Sebumlipid-ähnlichen Substanzen. Durch die gleichzeitige Verminderung des Tensidgehalts solcher Zubereitungen läßt sich der Schaden an der Hornschichtbarriere weiter begrenzen.

Dem Stand der Technik mangelt es allerdings an Zubereitungen, welche die Barrierefunktion und die Hydratation der Hornschicht positiv beeinflussen und die physikalischchemischen Eigenschaften der Hornschicht und insbesondere der Lamellen aus Interzellularlipiden stärken bzw. sogar wiederherstellen.

Aufgabe der vorliegenden Erfindung war es also, die Nachteile des Standes der Technik zu beseitigen. Insbesondere sollten hautpflegende Zubereitungen und Zubereitungen zur Reinigung der Haut zur Verfügung gestellt werden, welche die Barriereeigenschaften der Haut erhalten oder wiederherstellen, zumal dann, wenn die natürliche Regeneration der Haut nicht ausreicht. Sie sollen ferner zur Behandlung und Prophylaxe von Folgeschäden der Hautaustrocknung, beispielsweise Fissuren oder inflammatorischen oder allergischen Prozessen oder auch der Neurodermitis, geeignet sein. Aufgabe der vorliegenden Erfindung war es auch, stabile hautpflegende kosmetische und/oder dermatologische Mittel zur Verfügung zu stellen, welche die Haut vor Umwelteinflüssen wie Sonne und Wind schützen. Insbesondere sollte die Wirkung der Zubereitungen physiologisch, schnell und nachhaltig sein.

Diese Aufgaben werden überraschend und für den Fachmann nicht vorhersehbar gelöst durch
kosmetische oder dermatologische Zubereitungen mit einem Gehalt an unverzweigten, gesättigten und/oder ungesättigten, aliphatischen Fettsäuren, die eine Kettenlänge von C₂₂ bis C₆₀ haben, wobei die genannten Fettsäuren sowohl einzeln als auch im Gemisch vorliegen können.

Die erfindungsgemäßen Zubereitungen sind in jeglicher Hinsicht überaus befriedigende Präparate. Es war für den Fachmann nicht vorauszusehen gewesen, daß die erfindungsgemäßen Zubereitungen
- besser die Barriereeigenschaften der Haut erhalten oder wiederherstellen,
- besser der Hautaustrocknung entgegenwirken,
- besser gegen die Hautalterung wirken und
- die Haut besser vor Umwelteinflüssen schützen
als die Zubereitungen des Standes der Technik.

Vorteilhaft im Sinne der vorliegenden Erfindung sind insbesondere Fettsäuren bzw. Fettsäuregemische, welche durch Verseifung von Wachsen oder Wachsgemischen erhältlich sind. Die als Ausgangsprodukt verwendeten Wachse bzw. Wachsgemische können als natürliche Produkte unterschiedlich zusammengesetzt sein.

Die Verwendung von Wachsen und Wachsderivaten in kosmetischen oder dermatologischen Zubereitungen ist an sich bekannt. Wachse sind Fettsäureester, die in tierischen und pflanzlichen Produkten vorkommen, sich aber von den Fetten und Ölen dadurch unterscheiden, daß die zugrundeliegenden Fettsäuren nicht mit Glycerin, sondern mit höheren, primären, einwertigen Alkoholen verestert sind.

Vorteilhafte Fettsäuren bzw. Fettsäuregemische sind beispielsweise aus Bienenwachs, Chinawachs, Hummelwachs und anderen Insektenwachsen erhältlich.

Bienenwachs z. B. ist ein Ausscheidungsprodukt aus Drüsen der Honigbienen, das diese zum Bauen der Honigwaben verwenden. Gelbes (Cera flava), braunes oder rotes sogenanntes Rohwachs ist beispielsweise erhältlich, indem man die vom Honig durch Ausschleudern befreiten Waben schmilzt, die Schmelze von festen Verunreinigungen trennt und das so erhaltene Rohwachs erstarren läßt. Das Rohwachs kann durch Behandlung mit Oxidationsmitteln vollkommen weiß gebleicht werden (Cera alba).

Bienenwachs besteht aus dem in Alkohol leichtlöslichen Cerin, einem Gemisch aus Cerotinsäure CH₃(CH₂)₂₄COOH und Melissinsäure CH₃(CH₂)₂₈COOH sowie aus einem Myricin genannten Ester-Gemisch aus ca. 70 Estern von C₁₆- bis C₃₈-Säuren und C₂₄- bis C₃₆-Alkoholen. Als wesentliche Bestandteile von Bienenwachs finden sich Myricylpalmitat, Myricylcerotinat und Paraffin.

Auch andere Insektenwachse wie beispielsweise Hummelwachs oder Chinawachs sind im wesentlichen Mischungen verschiedener Ester. Chinawachs z. B. wird in China und Japan von der auf der chinesischen Esche lebenden Wachsschildlaus (Coccus ceriferus) und den Schildlausarten Ceroplastes ceriferus und Ericerus pela abgeschieden bzw. erzeugt. Es wird von den Bäumen abgekratzt und durch Umschmelzen in kochendem Wasser gereinigt. Hauptbestandteil des Chinawachses ist der Cerotinsäureester des Cerylalkohols.

Auch Fettsäuren bzw. Fettsäuregemische, welche aus Pflanzenwachsen erhältlich sind, sind vorteilhaft im Sinne der vorliegenden Erfindung. Vorzugsweise verwendbar sind Cuticularwachse niederer und höherer Pflanzen, Algen, Flechten, Moose und Pilze, wie beispielsweise Candelillawachs, Carnaubawachs, Japanwachs, Espartograswachs, Korkwachs, Reiswachs, Zuckerrohrwachs, Fruchtwachse, z. B. Apfelwachs, Blütenwachse, Blattwachse von Nadelhölzern, Kaffeewachs, Flachswachs, Sesamwachs, Jojobaöl und dergleichen mehr.

Candelillawachse z. B. sind bräunliche bis gelblichbraune, harte wachsartige Massen, welche in lipophilen Lösemitteln löslich sind. Candelillawachs enthält ungeradzahlige aliphatische Kohlenwasserstoffe (ca. 42 %), Ester (ca. 39 %), Wachssäuren und Wachsalkohole. Gewonnen werden kann es beispielsweise aus den zerkleinerten, fleischigen Blättern einer stachellosen Wolfsmilchart (Euphorbia cerifera) durch Auskochen mit wäßriger Schwefelsäure.

Carnaubawachs ist eine gelbliche, grünliche oder dunkelgraue Masse, welche in verschiedenen durch Auslese gewonnenen Qualitäten aus den Blättern der brasilianischen Fächerpalme Copernicia prunifera oder Carnaubapalme (Carnauba cerifera) gewonnen werden kann, indem beispielsweise der Wachsstaub von den angewelkten Wedeln gebürstet, geschmolzen, filtriert und nach dem Festwerden in Stücke gebrochen wird. Carnaubawachs kann durch Bleichmittel aufgehellt werden. Es enthält ca. 85 % Ester, jeweils etwa 2-3 % freie Wachssäuren (Carnauba-, Behen-, Lignocerin-, Melissin- und Cerotinsäure), langkettige Alkohole, Diole und gesättigte Kohlenwasserstoffe.

Japanwachs (auch: Japantalg oder Cera japonica) ist farbloses oder gelbliches, reines Pflanzenfett, das beispielsweise in Japan aus den Früchten eines baumförmigen Sumach-Gewächses (Rhus succedanea) durch Auskochen gewonnen werden kann. Hauptbestandteile des Japanwachses sind Palmitinsäureglycerinester sowie Ester der Japansäure (Heneicosandisäure, C₂₁H₄₀O₄), der Phellogensäure (Docosandisäure, C₂₂H₄₂O₄) und der Tricosandisäure (C₂₃H₄₄O₄).

Esparto-Wachs fällt als Nebenprodukt bei der Zellstoff- und Papierherstellung aus dem in Mittelmeerländern beheimateten Espartogras (Graminaceae) an. Es zu besteht zu ca. 15 bis 17 % aus Wachssäuren (z. B. Cerotin- und Melissinsäure), zu 20 bis 22 % aus Alkoholen und Kohlenwasserstoffen sowie zu 63 bis 65 % aus Estern.

Die erfindungsgemäßen Fettsäuren bzw. Fettsäuregemische sind beispielsweise erhältlich, indem man die Wachse oder Wachsgemische nach üblichen Verfahren verseift. Geringe Anteile an erfindungsgemäßen Fettsäuren bzw. Fettsäuregemischen sind aber auch durch Destillation aus den Wachsen oder Wachsgemischen erhältlich.

Zur Verseifung oder Hydrolyse werden die Wachse bzw. Wachsgemische in roher und/oder vorgereinigter - beispielsweise gebleichter - Form in an sich bekannter Weise, beispielsweise mit einer Base, vorzugsweise Alkalihydroxiden wie beispielsweise Natriumhydroxid (NaOH) und/oder Kaliumhydroxid (KOH) in Alkoholen, Wasser oder Gemischen davon, vorzugsweise aber in Alkoholen wie Methanol oder Ethanol, insbesondere bei pH 12 bis 14, verseift. Besonders bevorzugt ist die Verwendung von alkoholischer KOH-Lösung, insbesondere von ethanolischer Kalilauge.

Erfindungsgemäß vorteilhafte Insekten- und/oder Pflanzenwachshydrolysate mit einem Gehalt an unverzweigten, gesättigten und/oder ungesättigten, aliphatischen Fettsäuren, die eine Kettenlänge von C₂₂ bis C₆₀ haben, werden ebenfalls durch die saure Hydrolyse von Wachsen bzw. Wachsgemischen erhalten, beispielsweise durch die Hydrolyse mit anorganischen Mineralsäuren, wie z. B. Borsäure, Salzsäure, salpetriger und/oder Salpetersäure, schwefliger und/oder Schwefelsäure, den verschiedenen Phosphorsäuren und dergleichen mehr und/oder mit sauren Salzen der Elemente der 1. bis 3. Hauptgruppe und/oder mit organischen Säuren, wie z. B. Ameisensäure und/oder Essigsäure.

Durch die Spaltung erhält man im wesentlichen zwei Fraktionen, von denen die eine vorwiegend Alkohole und die andere vorwiegend Fettsäuren und Hydroxyfettsäuren enthält.

Eine Trennung der beiden Fraktionen voneinander erfolgt beispielsweise, indem zu der durch die alkalische Spaltung erhaltenen Mischung Wasser und ein mit Wasser nicht mischbares Lösungsmittel hinzugegeben wird und die unverseifbaren Anteile ausgeschüttelt werden. Vorteilhafte, mit Wasser nicht mischbare Lösungsmittel sind beispielsweise halogenfreie, z. B. Petrolether oder n-Heptan, oder halogenierte, insbesondere chlorierte Kohlenwasserstoffe.

Anschließend wird die wäßrige Phase mit einer Säure, beispielsweise mit einer Mineralsäure, vorzugsweise mit 1/10 bis 1/2-normaler Salzsäure, angesäuert und vorzugsweise so viel Wasser bzw. Säure zugegeben, bis ein pH-Wert von etwa 3 vorliegt.

Die sich abscheidende Fettsäure wird abfiltriert oder auch mit nicht wassermischbaren Lösungsmitteln wie halogenfreien, z. B. Petrolether oder n-Heptan, oder halogenierten, insbesondere chlorierten Kohlenwasserstoffen, z. B. Trichlormethan extrahiert.

Auf die beschriebene Weise erhält man beispielsweise aus einem Bienenwachsgemisch etwa 5 bis 65 Gew.-% - bezogen auf das Ausgangsgewicht - eines gereinigten Säuregemisches, insbesondere eines Hydroxyfettsäuregemisches und/oder Fettsäuregemisches. Das Verfahren liefert aus Bienenwachsgemischen vorzugsweise Fettsäurefraktionen, die zwischen 10 und 90 Gew.-% Fettsäuren enthalten, die besonders bevorzugt eine Kettenlänge von C₂₀ bis C₃₆ haben. Fettsäurefraktionen, die aus Pflanzenwachsen erhältlich sind, enthalten bevorzugt auch Fettsäuren mit einer deutlich höheren Kettenlänge.

Es ist erfindungsgemäß vorteilhaft, Insekten- und/oder Pflanzenwachshydrolysate mit einem Gehalt an unverzweigten, gesättigten und/oder ungesättigten, aliphatischen Fettsäuren, die eine Kettenlänge von C₂₂ bis C₆₀ haben, welche beispielsweise nach dem genannten Verfahren erhältlich sind, direkt in kosmetischen oder dermatologischen Zubereitungen einzusetzen. Vorzugsweise, wenngleich nicht zwingend kann sich an das beschriebene Verfahren eine weitere destillative Anreicherung der alkoholischen Komponenten anschließen.

Die erfindungsgemäßen kosmetischen oder dermatologischen Zubereitungen oder Formulierungen können wie üblich zusammengesetzt sein und zur Behandlung, der Pflege und der Reinigung der Haut und/oder der Haare und als Schminkprodukt in der dekorativen Kosmetik dienen. Entsprechend können sie, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Die erfindungsgemäßen Zubereitungen enthalten z. B. 0,001 bis 25 Gew.-%, bevorzugt 0,1 Gew.-% bis 15 Gew.-%, insbesondere aber 0,1 Gew.-% bis 10 Gew.-%, jeweils bezogen auf das Gesamtgewicht der Zubereitungen an den erfindungsgemäßen Wirkstoffen.

Günstig sind auch solche kosmetischen und dermatologischen Zubereitungen, die in der Form eines Sonnenschutzmittels vorliegen. Vorzugsweise enthalten diese neben einem oder mehreren erfindungsgemäßen Wirkstoffen mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die jeweils einzusetzenden Mengen an kosmetischen, dermatologischen oder medizinischen Trägerstoffen und Parfüm können in Abhängigkeit von der Art des jeweiligen Produktes vom Fachmann durch einfaches Ausprobieren leicht ermittelt werden.

Zubereitungen zur Behandlung und Pflege der Haut werden besonders bevorzugt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, die erfindungsgemäßen Wirkstoffe in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, die erfindungsgemäßen Wirkstoffe in wäßrige Systeme bzw. Tensidzubereitungen zur Reinigung der Haut und der Haare einzufügen.

Insbesondere können die erfindungsgemäßen kosmetischen und dermatologischen Zubereitungen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### Beispiel 1

| **W/O-Crème** | **I** | **II** | **III** |
|---|---|---|---|
| Paraffinöl | 10,00 | 10,00 | 10,00 |
| Ozokerit | 4,00 | 4,00 | 4,00 |
| Vaseline | 4,00 | 4,00 | 4,00 |
| pflanzliches Öl | 10,00 | 10,00 | 10,00 |
| PEG-7-hydriertes Ricinusöl | 4,00 | 4,00 | 4,00 |
| Octylmethoxycinnamat | 5,00 | 5,00 | 5,00 |
| Methylbenzyliden Campher | 2,50 | 2,50 | 2,50 |
| Tocopherolacetat | 1,00 | 1,00 | 1,00 |
| Magnesiumsulfat 7 H₂O | 0,70 | 0,70 | 0,70 |
| Glycerin | 3,00 | 3,00 | 3,00 |
| Bienenwachssäuren | 2,50 | - | - |
| Candelillawachssäuren | - | 2,50 | - |
| Reiswachssäuren | - | - | 2,50 |
| Parfüm, Konservierungsstoffe, Farbstoffe, Antioxidantien, Neutralisationsmittel | q.s. | q.s. | q.s. |
| Wasser, VES | ad 100,00 | ad 100,00 | ad 100,00 |

### Beispiel 2

| **W/O-Lotion** | **I** | **II** | **III** |
|---|---|---|---|
| Paraffinöl | 25,00 | 25,00 | 25,00 |
| Siliconöl | 2,00 | 2,00 | 2,00 |
| Ceresin | 1,50 | 1,50 | 1,50 |
| Vaseline | 9,00 | 9,00 | 9,00 |
| Wollwachsalkohol | 0,50 | 0,50 | 0,50 |
| Polyglyceryl-3-dioleat | 2,50 | 2,50 | 2,50 |
| Bienenwachssäuren | 1,00 | - | - |
| Hummelwachssäuren | - | 1,00 | - |
| Chinawachssäuren | - | - | 1,00 |
| Parfüm, Konservierungsstoffe, Farbstoffe, Antioxidantien, Neutralisationsmittel | q.s. | q.s. | q.s. |
| Wasser, VES | ad 100,00 | ad 100,00 | ad 100,00 |

### Beispiel 3

| **O/W-Lotion** | **I** | **II** | **III** |
|---|---|---|---|
| Paraffinöl | 5,00 | 5,00 | 5,00 |
| Isopropylpalmitat | 5,00 | 5,00 | 5,00 |
| Cetylalkohol | 2,00 | 2,00 | 2,00 |
| Bienenwachs | 2,00 | 2,00 | 2,00 |
| Ceteareth-20 | 2,00 | 2,00 | 2,00 |
| PEG-20-Glycerylstearat | 1,50 | 1,50 | 1,50 |
| Glycerin | 3,00 | 3,00 | 3,00 |
| Bienenwachssäuren | 1,00 | - | - |
| Apfelwachssäuren | - | 1,00 | - |
| Melissenblütenwachssäuren | - | - | 1,00 |
| Parfüm, Konservierungsstoffe, Farbstoffe, Antioxidantien, Neutralisationsmittel | q.s. | q.s. | q.s. |
| Wasser, VES | ad 100,00 | ad 100,00 | ad 100,00 |

### Beispiel 4

| **O/W-Crème** | **I** | **II** | **III** |
|---|---|---|---|
| Pflanzliches Öl | 10,00 | 10,00 | 10,00 |
| Cetylalkohol | 2,00 | 2,00 | 2,00 |
| Glycerinmonostearat | 1,50 | 1,50 | 1,50 |
| PEG-30-Glycerylstearat | 2,00 | 2,00 | 2,00 |
| Glycerin | 3,00 | 3,00 | 3,00 |
| Isopropylpalmitat | 5,00 | 5,00 | 5,00 |
| Carbopol 980 (neutralisiert) | 0,30 | 0,30 | 0,30 |
| Bienenwachssäuren | 1,50 | - | - |
| Sesamwachssäuren | - | 1,50 | - |
| Kaffeewachssäuren | - | - | 1,50 |
| Parfüm, Konservierungsstoffe, Farbstoffe, Antioxidantien, Neutralisationsmittel | q.s. | q.s. | q.s. |
| Wasser, VES | ad 100,00 | ad 100,00 | ad 100,00 |

### Beispiel 5

| **Salbe** | **I** | **II** | **III** |
|---|---|---|---|
| Vaseline | 36,00 | 36,00 | 36,00 |
| Ceresin | 10,00 | 10,00 | 10,00 |
| Zinkoxid | 4,00 | 4,00 | 4,00 |
| Pflanzliches Öl | 20,00 | 20,00 | 20,00 |
| Bienenwachssäuren | 0,2 | - | - |
| Espartawachssäuren | - | 0,2 | - |
| Carnaubawachssäuren | - | - | 0,2 |
| Parfüm, Konservierungsstoffe, Farbstoffe, Antioxidantien, Neutralisationsmittel | q.s. | q.s. | q.s. |
| Paraffinöl | ad 100,00 | ad 100,00 | ad 100,00 |

### Beispiel 6

| **Hautöl** | **I** | **II** | **III** |
|---|---|---|---|
| Cetylpalmitat | 3,00 | 3,00 | 3,00 |
| C₁₂₋₁₅- Alkylbenzoat | 2,00 | 2,00 | 2,00 |
| Polyisobuten | 10,00 | 10,00 | 10,00 |
| Squalan | 2,00 | 2,00 | 2,00 |
| Bienenwachssäuren | 0,50 | - | - |
| Korkwachssäuren | - | 0,50 | - |
| Japanwachssäuren | - | - | 0,50 |
| Parfüm, Konservierungsstoffe, Farbstoffe, Antioxidantien, Neutralisationsmittel | q.s. | q.s. | q.s. |
| Paraffinöl | ad 100,00 | ad 100,00 | ad 100,00 |

### Beispiel 7

| **Badeöl** | **I** | **II** | **III** |
|---|---|---|---|
| Paraffinöl | 20,00 | 20,00 | 20,00 |
| PEG-40-hydriertes Rizinusöl | 5,00 | 5,00 | 5,00 |
| Bienenwachssäuren | 0,50 | - | - |
| Sesamwachssäuren | - | 0,50 | - |
| Candelillawachssäuren | - | - | 0,50 |
| Parfüm, Konservierungsstoffe, Farbstoffe, Antioxidantien, Neutralisationsmittel | q.s. | q.s. | q.s. |
| Sojaöl | ad 100,00 | ad 100,00 | ad 100,00 |

### Beispiel 8

| **Lippenstift** | **I** | **II** | **III** |
|---|---|---|---|
| Ceresin | 8,00 | 8,00 | 8,00 |
| Bienenwachs | 14,00 | 14,00 | 14,00 |
| Carnaubawachs | 4,00 | 4,00 | 4,00 |
| Vaseline | 28,00 | 28,00 | 28,00 |
| Hydriertes Rizinusöl | 4,00 | 4,00 | 4,00 |
| Caprylic/Capric Triglyceride | 6,00 | 6,00 | 6,00 |
| Bienenwachssäuren | 0,20 | - | - |
| Reiswachssäuren | - | 0,20 | - |
| Candelillawachssäuren | - | - | 0,20 |
| Parfüm, Konservierungsstoffe, Farbstoffe, Antioxidantien, Neutralisationsmittel | q.s. | q.s. | q.s. |
| Paraffinöl | ad 100,00 | ad 100,00 | ad 100,00 |

### Beispiel 9

| **Pflegemaske** | **I** | **II** | **III** |
|---|---|---|---|
| PEG-50 Lanolin | 0,50 | 0,50 | 0,50 |
| Glycerylstearat | 2,00 | 2,00 | 2,00 |
| Sonnenblumenkernöl | 3,00 | 3,00 | 3,00 |
| Bentonit | 8,00 | 8,00 | 8,00 |
| Kaolin | 35,00 | 35,00 | 35,00 |
| Zinkoxid | 5,00 | 5,00 | 5,00 |
| Bienenwachssäuren | 1,00 | - | - |
| Chinawachssäuren | - | 1,00 | - |
| Japanwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 10

| **Liposomenhaltiges Gel** | **I** | **II** | **III** |
|---|---|---|---|
| Lecithin | 6,00 | 6,00 | 6,00 |
| Pflanzliches Öl | 12,50 | 12,50 | 12,50 |
| Hydrolysiertes Kollagen | 2,00 | 2,00 | 2,00 |
| Xanthan Gum | 1,40 | 1,40 | 1,40 |
| Butylenglycol | 3,00 | 3,00 | 3,00 |
| Bienenwachssäuren | 0,30 | - | - |
| Reiswachssäuren | - | 0,30 | - |
| Candelillawachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 11

| **Duschpräparat mit Rückfetter** | **I** | **II** | **III** |
|---|---|---|---|
| Cocoamidodiacetat | 10,00 | 10,00 | 10,00 |
| Natriumlaurylsulfat | 25,00 | 25,00 | 25,00 |
| Kalium Cocyl Hydrolysiertes Kollagen | 5,00 | 5,00 | 5,00 |
| Macadamianußöl | 5,00 | 5,00 | 5,00 |
| Natriumchlorid | 0,60 | 0,60 | 0,60 |
| Bienenwachssäuren | 0,30 | - | - |
| Hummelwachssäuren | - | 0,30 | - |
| Carnabauwachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 12

| **Seifenstück** | **I** | **II** | **III** |
|---|---|---|---|
| Na-Salz aus Talgfettsäuren | 60,00 | 60,00 | 60,00 |
| Na-Salz aus Kokosöl | 28,00 | 28,00 | 28,00 |
| Natriumchlorid | 0,50 | 0,50 | 0,50 |
| Bienenwachssäuren | 0,10 | - | - |
| Reiswachssäuren | - | 0,10 | - |
| Candelillawachssäuren | - | - | 0,10 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 13

| **Syndetseife** | **I** | **II** | **III** |
|---|---|---|---|
| Natriumlaurylsulfat | 30,00 | 30,00 | 30,00 |
| Natriumsulfosuccinat | 10,00 | 10,00 | 10,00 |
| Kaliumcocoyl hydrolysiertes Kollagen | 2,00 | 2,00 | 2,00 |
| Dimethicon Copolyol | 2,00 | 2,00 | 2,00 |
| Paraffin | 2,00 | 2,00 | 2,00 |
| Maisstärke | 10,00 | 10,00 | 10,00 |
| Talcum | 10,00 | 10,00 | 10,00 |
| Glycerin | 3,00 | 3,00 | 3,00 |
| Bienenwachssäuren | 1,00 | - | - |
| Sesamwachssäuren | - | 1,00 | - |
| Apfelwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 14

| **Haarpflegemittel** | **I** | **II** | **III** |
|---|---|---|---|
| TEA-Cocoyl hydrolysiertes Kollagen | 30,00 | 30,00 | 30,00 |
| Monoethanolaminlaurylsulfat | 25,00 | 25,00 | 25,00 |
| Mandelöl | 2,00 | 2,00 | 2,00 |
| Natriumchlorid | 1,00 | 1,00 | 1,00 |
| Bienenwachssäuren | 1,00 | - | - |
| Melissenblütenwachssäuren | - | 1,00 | - |
| Apfelwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 15

| **Pflegeshampoo** | **I** | **II** | **III** |
|---|---|---|---|
| Natriumlaurylsulfat | 34,00 | 34,00 | 34,00 |
| Dinatriumlaurylsulfosuccinat | 6,00 | 6,00 | 6,00 |
| Cocoamidopropylbetain | 10,00 | 10,00 | 10,00 |
| Glycoldistearat | 5,00 | 5,00 | 5,00 |
| Bienenwachssäuren | 0,20 | - | - |
| Melissenblütenwachssäuren | - | 0,20 | - |
| Apfelwachssäuren | - | - | 0,20 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 16

| **Haarkur** | **I** | **II** | **III** |
|---|---|---|---|
| Cetylalkohol | 5,00 | 5,00 | 5,00 |
| Caprylic/Capric Triglyceride | 3,00 | 3,00 | 3,00 |
| Petrolatum | 2,00 | 2,00 | 2,00 |
| Wollwachsalkohol | 0,50 | 0,50 | 0,50 |
| Bienenwachssäuren | 1,00 | - | - |
| Reiswachssäuren | - | 1,00 | - |
| Apfelwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 17

| **Haarspülung** | **I** | **II** | **III** |
|---|---|---|---|
| Cocoamidopropylbetain | 5,00 | 5,00 | 5,00 |
| Cetylalkohol | 2,00 | 2,00 | 2,00 |
| Propylenglycol | 2,00 | 2,00 | 2,00 |
| Citronensäure | 0,30 | 0,30 | 0,30 |
| Bienenwachssäuren | 1,00 | - | - |
| Sesamwachssäuren | - | 1,00 | - |
| Carnaubawachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 18

| **Haarfestiger** | **I** | **II** | **III** |
|---|---|---|---|
| Polyvinylpyrrolidon/Vinylacetat/Vinylpropionat-Copolymer | 5,00 | 5,00 | 5,00 |
| Ethanol | 45,00 | 45,00 | 45,00 |
| Bienenwachssäuren | 1,00 | - | - |
| Candelillawachssäuren | - | 1,00 | - |
| Apfelwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 19

| **Frisiercrème** | **I** | **II** | **III** |
|---|---|---|---|
| Vaseline | 4,00 | 4,00 | 4,00 |
| Cetearylalkohol | 4,00 | 4,00 | 4,00 |
| PEG-40-hydriertes Rizinusöl | 2,00 | 2,00 | 2,00 |
| Isopropylpalmitat | 5,00 | 5,00 | 5,00 |
| Citronensäure | 1,00 | 1,00 | 1,00 |
| Bienenwachssäuren | 0,30 | - | - |
| Kaffeewachssäuren | - | 0,30 | - |
| Apfelwachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 20

| **Rasierschaum** | **I** | **II** | **III** |
|---|---|---|---|
| Stearinsäure | 7,00 | 7,00 | 7,00 |
| Natriumlaurylsulfat | 3,00 | 3,00 | 3,00 |
| Stearylalkohol | 1,00 | 1,00 | 1,00 |
| Glycerin | 5,00 | 5,00 | 5,00 |
| Triethanolamin | 3,60 | 3,60 | 3,60 |
| Bienenwachssäuren | 0,30 | - | - |
| Korkwachssäuren | - | 0,30 | - |
| Chinawachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 21

| **Fußcrème** | **I** | **II** | **III** |
|---|---|---|---|
| Soluan 5 | 2,00 | 2,00 | 2,00 |
| Methylsalicylat | 5,00 | 5,00 | 5,00 |
| Caprylic/Capric Triglyceride | 10,00 | 10,00 | 10,00 |
| Stearinsäure | 5,00 | 5,00 | 5,00 |
| Cetylalkohol | 1,00 | 1,00 | 1,00 |
| Glycerin | 2,00 | 2,00 | 2,00 |
| Dimethicon | 1,00 | 1,00 | 1,00 |
| Carbopol 984 | 0,50 | 0,50 | 0,50 |
| Triethanolamin | 1,50 | 1,50 | 1,50 |
| Bienenwachssäuren | 1,00 | - | - |
| Kaffeewachssäuren | - | 1,00 | - |
| Apfelwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 22

| **Aerosolspray** | **I** | **II** | **III** |
|---|---|---|---|
| Octyldodecanol | 0,50 | 0,50 | 0,50 |
| Bienenwachssäuren | 0,20 | - | - |
| Kaffeewachssäuren | - | 0,20 | - |
| Apfelwachssäuren | - | - | 0,20 |
| Parfüm, Konservierungsstoffe, Farbstoffe, Antioxidantien, Neutralisationsmittel | q.s. | q.s. | q.s. |
| Ethanol | ad 100,00 | ad 100,00 | ad 100,00 |

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 23

| **Pumpspray** | **I** | **II** | **III** |
|---|---|---|---|
| PEG-40-Hydriertes Rizinusöl | 2,00 | 2,00 | 2,00 |
| Glycerin | 1,00 | 1,00 | 1,00 |
| Bienenwachssäuren | 0,20 | - | - |
| Reiswachssäuren | - | 0,20 | - |
| Sesamwachssäuren | - | - | 0,20 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 24

| **Roll-on-Gel** | **I** | **II** | **III** |
|---|---|---|---|
| 1,3-Butylenglycol | 2,00 | 2,00 | 2,00 |
| PEG-40-Hydriertes Rizinusöl | 2,00 | 2,00 | 2,00 |
| Hydroxyethylcellulose | 0,50 | 0,50 | 0,50 |
| Bienenwachssäuren | 0,30 | - | - |
| Chinawachssäuren | - | 0,30 | - |
| Japanwachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 25

| **Roll-on-Emulsion** | **I** | **II** | **III** |
|---|---|---|---|
| Tricetearethphosphat | 0,30 | 0,30 | 0,30 |
| Octyldodecanol | 2,00 | 2,00 | 2,00 |
| C₁₂₋₁₅-Alkylbenzoat | 2,00 | 2,00 | 2,00 |
| C₁₀₋₃₀-Alkylacrylat | 0,15 | 0,15 | 0,15 |
| Bienenwachssäuren | 0,30 | - | - |
| Melissenblütenwachssäuren | - | 0,30 | - |
| Apfelwachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 26

| **Wachsstift** | **I** | **II** | **III** |
|---|---|---|---|
| Hydriertes Rizinusöl | 5,00 | 5,00 | 5,00 |
| Bienenwachs | 6,00 | 6,00 | 6,00 |
| Ceresin | 30,00 | 30,00 | 30,00 |
| C12-15-Alkylbenzoat | 17,00 | 17,00 | 17,00 |
| Bienenwachssäuren | 0,50 | - | - |
| Reiswachssäuren | - | 0,50 | - |
| Sesamwachssäuren | - | - | 0,50 |
| Parfüm, Konservierungsstoffe, Farbstoffe, Antioxidantien, Neutralisationsmittel | q.s. | q.s. | q.s. |
| Octyldodecanol | ad 100,00 | ad 100,00 | ad 100,00 |

### Beispiel 27

| **Emulsion-Make-up** | **I** | **II** | **III** |
|---|---|---|---|
| Caprylic/Capric Triglyceride | 7,50 | 7,50 | 7,50 |
| Lecithin | 2,00 | 2,00 | 2,00 |
| Distärkephosphat | 1,00 | 1,00 | 1,00 |
| Dimethicon | 0,50 | 0,50 | 0,50 |
| Glycerin | 1,50 | 1,50 | 1,50 |
| Magnesiumsilikat | 1,00 | 1,00 | 1,00 |
| Glimmer | 1,00 | 1,00 | 1,00 |
| Eisenoxide | 1,00 | 1,00 | 1,00 |
| Titandioxid | 2,50 | 2,50 | 2,50 |
| Talkum | 5,00 | 5,00 | 5,00 |
| Tapiokastärke | 0,25 | 0,25 | 0,25 |
| Bienenwachssäuren | 0,30 | - | - |
| Reiswachssäuren | - | 0,30 | - |
| Sesamwachssäuren | - | - | 0,30 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 28

| **Maskara** | **I** | **II** | **III** |
|---|---|---|---|
| Stearinsäure | 5,00 | 5,00 | 5,00 |
| Lanolin | 5,00 | 5,00 | 5,00 |
| Magnesiumaluminiumsilikat | 3,00 | 3,00 | 3,00 |
| Cyclomethicon | 0,50 | 0,50 | 0,50 |
| Pigmentmischung | 5,00 | 5,00 | 5,00 |
| Bienenwachssäuren | 1,00 | - | - |
| Candelillawachssäuren | - | 1,00 | - |
| Sesamwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 29

| **Maskara, wasserfest** | **I** | **II** | **III** |
|---|---|---|---|
| Lanolin | 7,00 | 7,00 | 7,00 |
| Aluminiumstearat | 2,00 | 2,00 | 2,00 |
| Bienenwachs | 5,00 | 5,00 | 5,00 |
| Cyclomethicon | 5,00 | 5,00 | 5,00 |
| Polyvinylpyrrolidon Eicosen Copolymer | 1,00 | 1,00 | 1,00 |
| Pigmentmischung | 10,00 | 10,00 | 10,00 |
| Bienenwachssäuren | 2,00 | - | - |
| Candelillawachssäuren | - | 2,00 | - |
| Carnaubawachssäuren | - | - | 2,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

### Beispiel 30

| **Lidschatten** | **I** | **II** | **III** |
|---|---|---|---|
| Acetylierter Lanolinalkohol | 2,00 | 2,00 | 2,00 |
| Cetylacetat | 0,50 | 0,50 | 0,50 |
| Paraffinöl | 5,00 | 5,00 | 5,00 |
| Polysorbat 80 | 2,00 | 2,00 | 2,00 |
| Cyclomethicon | 2,00 | 2,00 | 2,00 |
| Glycerylmonostearat | 4,50 | 4,50 | 4,50 |
| Stearinsäure | 13,50 | 13,50 | 13,50 |
| Pigmentmischung | 10,00 | 10,00 | 10,00 |
| Bienenwachssäuren | 1,00 | - | - |
| Reiswachssäuren | - | 1,00 | - |
| Sesamwachssäuren | - | - | 1,00 |
| Parfüm, Konservierungsstoffe, Farbstoffe, Antioxidantien, Neutralisationsmittel | q.s. | q.s. | q.s. |
| Wasser | ad 100,00 | ad 100,00 | ad 100,00 |

## Patentansprüche

1. Kosmetische oder dermatologische Zubereitungen mit einem Gehalt an unverzweigten, gesättigten und/oder ungesättigten, aliphatischen Fettsäuren, die eine Kettenlänge von C₂₂ bis C₆₀ haben, wobei die genannten Fettsäuren sowohl einzeln als auch im Gemisch vorliegen können.

2. Kosmetische oder dermatologische Zubereitungen nach Anspruch 1, dadurch gekennzeichnet, daß zusätzlich kosmetische oder dermatologische Hufs-, Zusatz- und/oder Wirkstoffe enthalten sind.

3. Kosmetische oder dermatologische Zubereitungen nach einem der Ansprüche 1 oder 2, dadurch gekennzeichnet, daß die Fettsäuren bzw. die Fettsäuregemische aus Insektenwachsen erhältlich sind.

4. Kosmetische oder dermatologische Zubereitungen nach einem der Ansprüche 1 oder 2, dadurch gekennzeichnet, daß die Fettsäuren bzw. die Fettsäuregemische aus Pflanzenwachsen erhältlich sind.

5. Kosmetische oder dermatologische Zubereitungen nach einem der vorhergehenden Ansprüche dadurch gekennzeichnet, daß die Fettsäuren eine Kettenlänge von C₂₂ bis C₃₄ haben.

6. Kosmetische oder dermatologische Zubereitungen mit einem Gehalt an Insekten- und/oder Pflanzenwachshydrolysaten, welche einen Gehalt an unverzweigten, gesättigten und/oder ungesättigten, aliphatischen Fettsäuren haben, die eine Kettenlänge von C₂₂ bis C₆₀ aufweisen, wobei die genannten Fettsäuren sowohl einzeln als auch im Gemisch vorliegen können.

7. Verwendung von kosmetischen oder dermatologischen Zubereitungen mit einem Gehalt an unverzweigten, gesättigten und/oder ungesättigten, aliphatischen Fettsäuren, die eine Kettenlänge von C₂₂ bis C₆₀ haben, wobei die genannten Fettsäuren sowohl einzeln als auch im Gemisch vorliegen können, zur Prophylaxe und Behandlung von schädigenden und/oder oxidativen Einwirkungen auf die Haut oder die Haare.

8. Verwendung von kosmetischen oder dermatologischen Zubereitungen mit einem Gehalt an unverzweigten, gesättigten und/oder ungesättigten, aliphatischen Fettsäuren, die eine Kettenlänge von C₂₂ bis C₆₀ haben, wobei die genannten Fettsäuren sowohl einzeln als auch im Gemisch vorliegen können, zur Stärkung der Barrierefunktion der Haut.
